# EUROPÄISCHE PATENTSCHRIFT

(11) **EP 3 067 684 B1**
(45) Veröffentlichungstag und Bekanntmachung des Hinweises auf die Patenterteilung: **24.02.2021**
(21) Anmeldenummer: 16157981.8
(22) Anmeldetag: 01.03.2016
(51) Int. Cl.: G01N 11/14

(54) **DREHRHEOMETER**
ROTATING RHEOMETER
RHEOMETRE ROTATIF

(30) Priorität: 11.03.2015 AT 501962015
(43) Veröffentlichungstag der Anmeldung: 14.09.2016
(73) Patentinhaber: Anton Paar GmbH, 8054 Graz-Straßgang (AT)
(72) Erfinder: Romirer, Richard, 8054 Graz (AT); Schütz, Denis, 8010 Graz (AT); Narnhofer, Matthias, 8010 Graz (AT); Flecker, Andreas, 8570 Voitsberg (AT)
(74) Vertreter: Wildhack & Jellinek

(56) Entgegenhaltungen:
- EP-A2- 1 750 114
- DE-A1- 3 722 862
- GB-A- 2 180 343
- JP-A- S60 179 632
- US-A1- 2002 178 842
- US-A1- 2005 132 783
- US-B1- 6 167 752

## Beschreibung

Die Erfindung betrifft ein Drehrheometer zur Vermessung von pulvrigen oder granularen Materialien, umfassend einen Messbehälter zur Aufnahme des Messguts und eine Abdeckung für den Messbehälter und einen von einer Messwelle angetriebenen Messkörper, der im Behälter relativ zu diesem rotierbar ist, wobei die Messwelle berührunglos durch die Abdeckung geführt ist und mit einem außerhalb des Messbehälters angeordneten Messsystem zur Auswertung der von der Messwelle abgenommenen Messwerte.

Der Erfindung liegt die Aufgabe zugrunde, ein einfach aufgebautes Drehrheometer zur Vermessung von Pulvern und/oder Schüttgut und/oder granularem Material zu erstellen und sowohl den Benutzer als auch empfindliche Geräteteile vor aus- bzw. auftretenden Staub zu schützen.

Rheometer zur rheologischen Charakterisierung von Pulvern sind bekannt. Dabei wird ein Messkörper in einem Behälter mit dem zu untersuchenden Schüttgut bzw. Pulver bzw. Paritkelmaterial rotierend bzw. rotierend oszillierend bewegt, teils auch in Kombination mit einer Linearbewegung des Messkörpers, insbesondere auf- oder abwärts in der Probe. Das durch die Probe der Bewegung entgegenwirkende Drehmoment und/oder die dabei auftretede Normalkraft werden gemessen. Dabei werden unterschiedlichste Ausführungsformen von Rheometern oder Viskosimetern verwendet, die entweder als universal einsetzbare Rheometer mit speziellen Pulvermesszellen ausgestattet werden oder es werden direkt für die Anwendung hergestellte Geräte verwendet, oft auch mit Zusatzauswertemöglichkeiten wie beispieslweise Packungsdichte und/oder Gewicht der Prüfsubstanz usw.
Die Pulver können dabei sowohl in einem fluidisierten bzw. dynamischen Zustand als auch im unfluidisieren bzw. statischen Zustand gemessen werden. Auch Anordnungen, bei denen das Pulver unter Druck vorgepresst oder auch während der Messung mit Druck beaufschlagt wird, sind bekannt. Die Probenvorbereitung durch Fluidisierung ermöglicht das "Löschen des Pulvergedächtnis" z.B. von Einfülleffekten, Caking, usw., mit anschließender statischer Pulvercharakterisierung. Im dynamischen Fall soll während der Messung das Pulver in fluidisiertem Zustand gehalten werden können.

Die JPH 08271400 A zeigt ein System, das die Eindringtiefe eines konischen Rotors und das auftretende Drehmoment zur Charakterisierung des Pulvers misst und gleichzeitig mit Mitteln zur Vibration des Messbechers eine Fluidisierung des Pulvers im Messbecher vornimmt.

Die JP 2007040770 zeigt ein System mit der Möglichkeit, einen Messkörper rotierend durch ein Pulver in einem Messbecher zu bewegen. Eine Anordnung mit Druckluft, die das Pulver durchströmt, ermöglicht dabei die Fluidisierung des Pulvers.

Aus der US 2002/178842 A1 ist ein Viskosimeter bekannt, bei dem sich ein äußeres Messteil und ein inneres Messteil relativ zueinander drehen können, die gemeinsam in die Probe getaucht werden. Die Lagerung der Messwelle im Deckel erfolgt hier nicht mit einem Kugellager, der Spritzschutz dient dem Verhindern von Ablagerungen und Verschmutzung am Lager. Die Durchführung der Zu- und Ableitung von Druckluft dient der Entnahme von Gasen aus dem Raum über dem Fluid Die offenen Zu- und Ableitungen sind mit dem gleichen Spritzschutz abgedeckt, wie die Lager der Messwelle.

Eine Fluidisierung kann aber auch durch Vibration hervorgerufen werden, beispielsweise mittels Piezoelementen, Ultraschall oder Unwuchtmotor. Gleichzeitig kann diese Vibration genutzt werden, um ein bestimmtes Pulverlevel im Messbecher einzustellen. Durch die Fluidisierung und das dabei durchgeführte "Löschen des Pulvergedächtnises" wird eine anwenderunabhängige Messung sichergestellt.

Allen Geräten ist gemeinsam, dass der Messkörper relativ zum Pulver bewegt wird. Diese Bewegung kann rotierend oder rotierend oszillierend ausgeführt werden. Dabei kann entweder der Messbecher rotiert werden und der Messkörper mit Mitteln zur Drehmomentbestimmung ausgestattet werden oder der Messkörper wird rotiert und die auftretenden Drehmomente werden beispielsweise mit einem an der Messwelle angreifenden Messmotor bestimmt.

Gegebenenfalls erfolgt die Bewegung noch kombiniert mit einem linearen Vorschub entlang der Rotationsachse, der dann zu einer schraubförmigen Bewegung des Messkörpers im Pulver führt.

Der Messmotor eines Rheometers kann dann dazu genutzt werden, die am Messkörper auftretenden Normalkräfte und/oder Drehmomente aufzuzeichnen. Die Auswertung erfolgt analog zum Rheometer basierend auf diesen Messwerten, aber auch phänomenologische Auswertungen können durchgeführt werden, z.B. die Angabe der sogenannten Flow Energy (Einheit: Joule) als Integral des Drehmoments über den Drehwinkel und/oder der Normalkraft über den Verfahrweg gemessen beim Verfahren des Messkörpers im Pulver.

Um mit einem Rheometer hochgenaue Messungen ausführen zu können, muss die Rotation des Messkörpers möglichst reibungsfrei stattfinden. Daher werden an die Lagerung der Messwelle, die den Messkörper trägt, hohe Anforderungen gestellt, im Regelfall werden möglichst reibungsfreie Kugellager, Magnetlager, Fluidlager und dgl. verwendet. Die auftretenden Normalkräfte können auf unterschiedlichste Weise gemessen werden. Die Messzelle kann beispielsweise als "Wiegezelle" am Messbecher ausgeführt werden, oder auch am Messmotor oder an der Messkörperachse können Messeinheiten angeordnet sein. Rheometer können beispielsweise auch spezielle Luftlager mit integrierter Normalkraftmessung (AT 404192) besitzen.

Die Messwelle wird also möglichst reibungsfrei, insbesondere berührungsfrei gelagert, vom antreibenden Motor zum Messkörper geführt.

Bekannt sind auch spezielle Rheometerausführungen, um Messwellen, Lager und Antriebsmotor vor agressiven Gasen und/oder Feuchtigkeit zu schützen. Dies kann einerseits durch möglichst reibungsfreie Dichtungen erfolgen. Stopfbuchsen finden beispielsweise Verwendung, um das Eindringen aggressiver Gase in den Messmotor zu verhindern; auch Labyrinthdichtungen sind dafür bekannt.

Um mit einem derartigen Rheometer Pulvermessungen universell und insbesondere auch für fluidisierte Pulver durchführen zu können, reicht der Einsatz einer einzigen derartigen Dichtung nicht aus. Das empfindliche, zumeist elektronische Messsystem muss vor allem durch spezielle Vorkehrungen vor dem Eindringen von Staub geschützt werden. Ferner soll das Austreten gesundheitsschädlicher Pulver aus der Messzelle bzw. dem Messbereich hintangehalten werden.

Ist das Pulver in einem fluidisierten Zustand bildet sich über dem Pulverbett ein Aerosol aus, welches aus dem Feinanteil des Pulvers besteht. Zudem werden größere, schwerere Pulverteilchen bei der Fluidisierung mit hoher kinetischer Energie in Richtung der Dichtung bzw. durch die Durchführung der Messwelle geschleudert.

Ein Ziel der Erfindung ist die Erstellung eines genaue Messwerte liefernden Pulverrheometers mit adäquatem Staubschutz. Ohne ausreichenden Staubschutz ist die Anwendung zumeist auf sehr spezielle Pulver beschränkt. Pulver, die sich nur schlecht fluidisieren lassen und bei der Durchleitung von Gasen große, platzende Pulverblasen bilden, sind im Regelfall von der Untersuchung ausgeschlossen. Die Pulverteilchen erhalten durch diese platzenden Blasen sehr hohe kinetische Energien und werden in alle Richtungen und auch aus dem Becherinnenraum bzw. Messbehälter hinaus gegen die Messachse bzw. -welle und/oder Lager des Messmotors bzw. die Bauteile für die Drehmomentmessung und/oder die Mittel zur Normalkraftmessung geschleudert.

Wenn die Pulverteilchen sehr fein sind, wird eine Labyrinthdichtung nicht ausreichend abdichten. Haben die Pulverkomponenten zu hohe Energien, kann eine Labyrinthdichtung durch eindringende Teilchen befüllt werden und versagen.

Kugellager, die gegen den Eintrag von Pulverstäuben robuster sind, können bei der Messung von feinen, staubbildenen Pulvern Schaden nehmen. Zudem lassen sich mit diesen Anordnungen die auftretenden Drehmomente nicht hinreichend genau bestimmen.

Der Erfindung liegt auch die wesentliche Aufgabe zugrunde, einen Staubschutz für ein Rheometer zur Verfügung zu stellen, sodass universelle Pulver unterschiedlichster Zusammensetzung, Größenverteilung und Fluidisierbarkeit vermessen werden können. Zusätzlich sollen aus Gründen der Bedienersicherheit die Pulver nicht in die Geräteumgebung freigesetzt werden können. Damit können dann auch Pulver mit aggressiven Komponenten, die für die Bedienungsperson ein gesundheitliches Risiko darstellen, untersucht werden.

Eine weitere Aufgabe der Erfindung ist es also, ein Pulvermesssystem bzw. Rheometer zur Vefügung zu stellen, das ein Verwirbeln und Freisetzen von Pulveranteilen in die Umgebungsluft verhindert.

Erfindungsgemäß ist ein Drehrheometer nach Anspruch 1. Erfindungsgemäß wird somit eine Kombination aus zumindest zwei Dichtprinzipien bzw. Dichtvarianten eingesetzt, um ein Austreten von Pulver aus dem Messbehälter bzw. ein Eindringen von Pulverbestandteilen in das Rheometer, insbesondere in die Auswerteelektronik, zu verhindern.

Dadurch wird verhindert, dass im Bereich von Durchführungen und Lagern der Messwelle Pulveranlagerungen entstehen und damit zusätzliche Reibungskräfte. Dies erhöht die Wiederholbarkeit der Messung und sorgt bei der Verwendung von beispielsweise Luftlagern im Rheometer, zu höchster Messgenauigkeit hinsichtlich der auftretenden Drehmomente.

Es ist erfindungsgemäß vorgesehen, einen Staubschutz für ein Rheometer in Form einer Kombination einer Fluiddichtung und einer geometrischer Sperre bei berührungsloser Durchführung der Messwelle durch die Abdeckung des Messbehälters vorzusehen. Das verwendete Dichtfluid ist bevorzugt dasselbe Gas, das für die Fluidisierung des Pulvers verwendet wird. Die geometrische Sperre hält mit feinen Kanälen bzw. mit feinen geometrischen Strukturen oder auch ineinandergreifenden Verzahnungen nach Art einer Labyrinthdichtung für das Dichtfluid die feinen Pulverteilchen im Aerosol zurück. Das Problem, dass die Verwendung von nur einer Art einer Dichtung nicht ausreicht, um die bei Pulvermessungen auftretenden Komplikationen zu vermeiden, wurde erfindungsgemäß gelöst. Für den Fachmann kam diese Lösung unerwartet, da der Einsatz der Dichtungen eine Beeinträchtigung der Qualität der Messwerte erwarten ließ.

Bevorzugte Ausführungsformen sind in den abhängigen Ansprüchen angegeben.

Im Folgenden wird die Erfindung beispielsweise anhand der Zeichnungen näher erläutert.

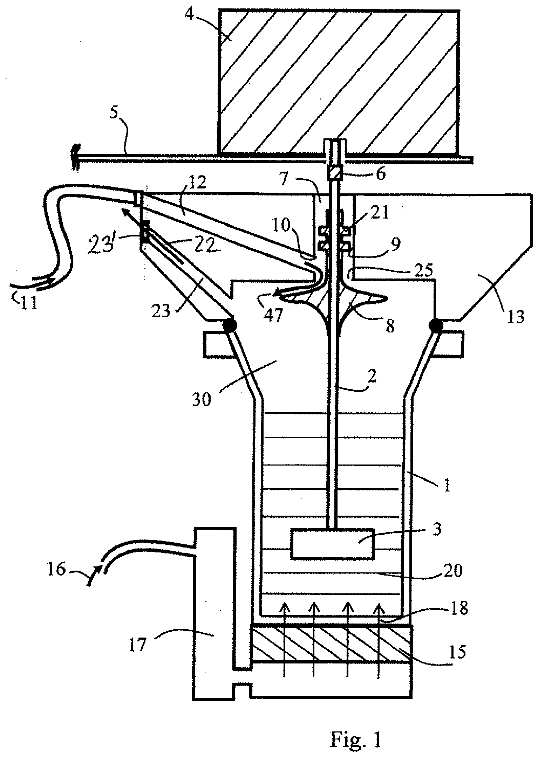

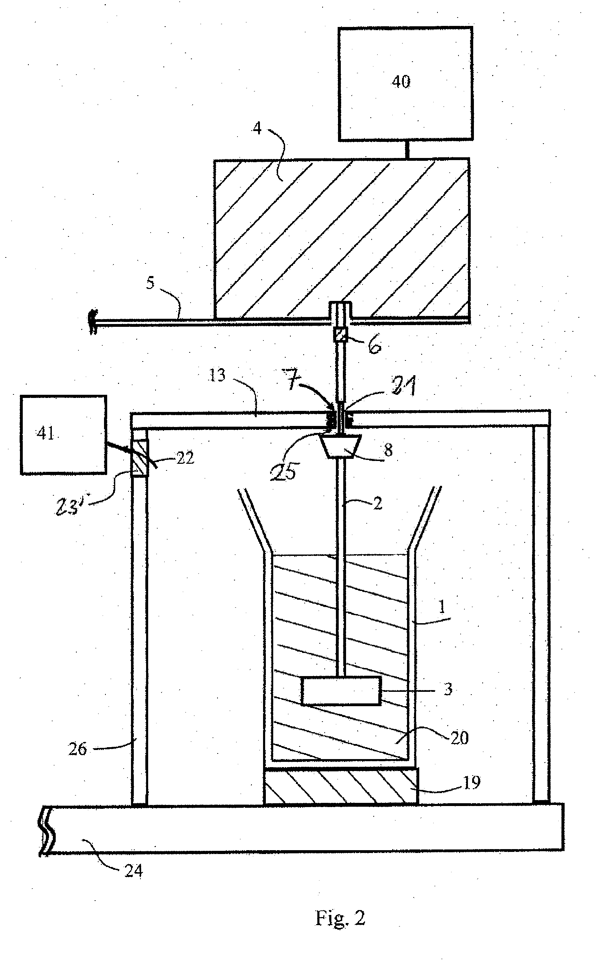

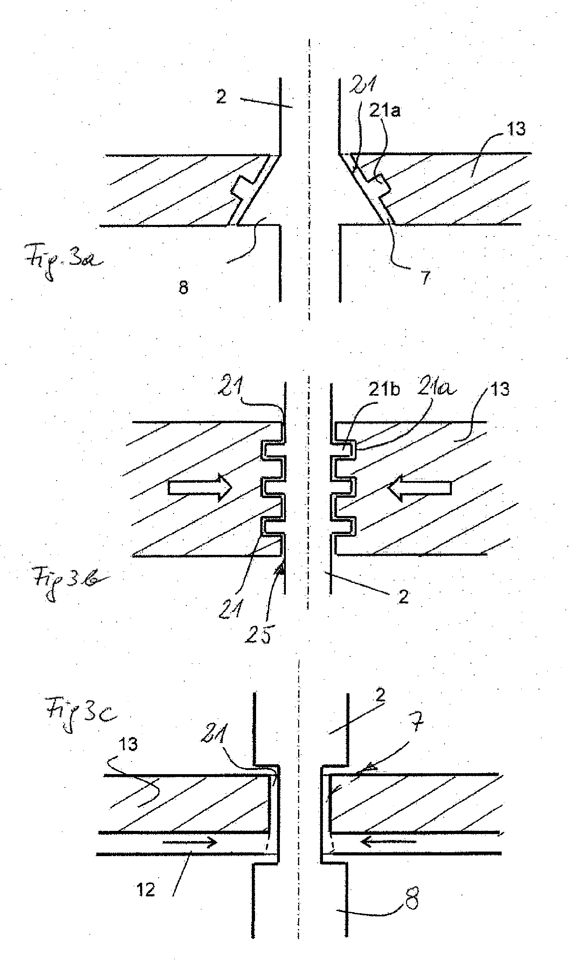

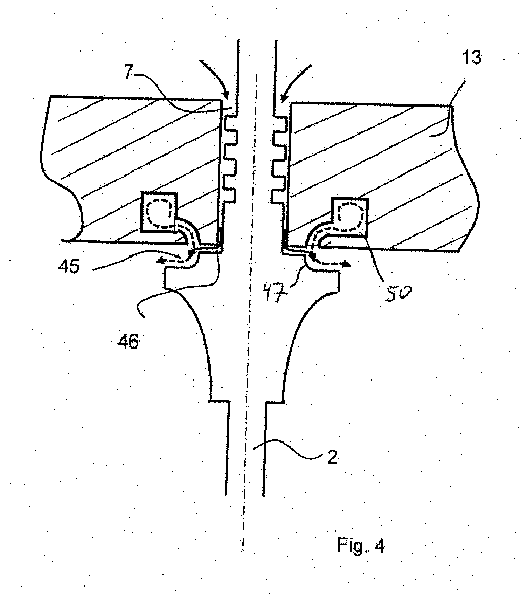

Fig. 1 zeigt einen schematischen Schnitt durch ein erfindungsgemäßes Rheometer mit einem Messbehälter mit einem aufgesetzten Deckel. Fig. 2 zeigt ein Rheometer, bei dem der Messbehälter mit einer Umhüllung bzw. einem Hüllgehäuse umgeben ist. Fig. 3a, 3b und 3c zeigen unterschiedlich gestaltete Durchführungen der Messwelle durch eine Abdeckung des Mesbehälters oder des Hüllgehäuses. Fig. 4 zeigt eine Ausführungsform einer Fluiddichtung.

Die Fig. 1 zeigt schematisch eine Ausführungsform eines Pulverrheometers mit einem zylindrischen Messbehälter 1, der gleichzeitig auch der Fluidisierung des zu untersuchenden Pulvers 20 mit z.B. über eine Leitung 17 gemäß Pfeil 16 zugeführter Druckluft dient. Die Druckluft oder ein anderes Arbeitsgas wird über die Leitung 17 und einen Diffusor 15 gemäß Pfeilen 18 in den Messbehälter 1 gepumpt. Es können unterschiedliche Materialien für die Fritten des Diffusors 15 eingesetzt werden, die für eine an das jeweilige zu vermessende Pulver 20 angepasste Verwirbelung sorgen.

Auf die Messzelle bzw. den Messbehälter 1 ist eine Abdeckung 13 gesetzt, die z.B. festgeschraubt oder festgeklemmt werden kann. Neben einer Kombination von Schrauben und einem Dichtring 14 sind auch Schraubverbindungen, Schnappverschlüsse oder Flanschverbindungen denkbar. Der Messbehälter 1 kann auch mehrteilig ausgeführt werden, um diesen einfach aus dem Rheometer bzw. dem Fluid-System entfernen, befüllen, wiegen und / oder reinigen zu können.

Durch eine Durchführung 7 in der Abdeckung 13 des Messbehälters 1 ist eine Messwelle 2 durchgeführt, die in ihrem unteren, in den Messbehälter 1 ragenden Endbereich einen Messkörper 3 trägt. Dieser Messkörper 3 ragt in das Pulver 20 und ist über eine Kupplung 6 mit einem Antriebs- bzw. Messmotor 4 verbunden, der von einem Träger 5 oberhalb des Messbehälters 1 abgestützt ist. Der Antriebs- bzw. Messmotor 4 rotiert die Messwelle 2 im Pulver 20.

Anordnungen betreffend die Messung von auftretenden Normalkräften und/oder Drehmomenten bzw. Antriebe für die Rotation des Antriebs- bzw. Messmotors 4 und allfälliges Anheben und Absenken des Messkörpers 3 sind nicht dargestellt. Eine Auswerteeinheit 40 für die abgenommenen Messwerte ist in Fig. 2 schematisch dargestellt. Alle diese Einheiten sind dem Fachmann ebenso wie der grundlegende Aufbau von Rheometern bekannt.

Die Abdeckung 13 ist im vorliegenden Fall einstückig ausgeführt und besitzt in der Mitte eine Bohrung bzw. Durchführung 7 zur Aufnahme und Durchführung der Messachse bzw. -welle 2, die den Messkörper 3 trägt. Die Messwelle 2 trägt im vorliegenden Fall Bauteile der geometrischen Dichtung. Die Abdeckung 13 weist durch z.B. Drehen oder Fräsen erstellte Bauteil der geometrischen Dichtung auf bzw. trägt Teile der geometrischen Dichtung. Im vorliegenden Fall ist zwischen der Messwelle 2 und der Abdeckung 13 eine Labyrinthdichtung abgebildet, die einen Spalt 9 besitzt, der für eine Luftführung ausgenutzt werden kann. Gegebenefalls können diese Labyrinthdichtung auch in der Abdeckung 13 durch Ausbildung einzelner Nuten eingeschniten werden. Vorteilhaft tragen sowohl die Abdeckung 13 als auch die Messwelle 2 Teile des Labyrinthprofils. Insbesondere hier ist es von Vorteil, wenn die Abdeckung 13 mehrteilig ist. Für eine einstückige Ausführung der Abdeckung 13 ist es auch wichtig, dass die Messwelle 2 durch die Abdeckung 13 durchgeführt werden kann, und dass zur Aufnahme auswechselbarer Messkörper 3 und der Messwelle 2 ein Koppelsystem 6 zum Messmotor 4 vorgesehen ist. Über einen in die Abdichtung bzw. Durchführung 7 unterhalb der geometrischen Dichtung mündenden Lufteinlass 10, der beispielsweise an die Druckluftzufuhr des Rheometers für die Pulverfluidisierung angeschlossen wird, kann die gesamte geometrische Dichtung mit Luft gespült werden, indem durch diese Luft eingeblasen oder angesaugt wird. Dabei sind die Luftzufuhr und die Abdichtung bevorzugt zentrisch symmetrisch ausgeführt, um die Messung möglichst wenig zu beeinflussen.

Der durch den zylindrischen Messbecher 1 und die Abdeckung 13 begrenzte Messraum 30 wird durch einen Abluftkanal 23 entlüftet. Auch hier kann eine symmetrische Führung der Luftströme erfolgen. Am ausströmseitigen Ende kann der Abluftkanal 23 durch einen auswechsel- und reinigbaren Filter 45 verschlossen sein, der das Austreten von verwirbelten Pulverteilen verhindert.

Ein mechanischer Spritzschutz bzw. Schirm 8 verhindert als geometrische Dichtung das direkte Auftreffen von Pulverteilchen mit hoher kinetischer Energie im Bereich um die Durchführung 7 der Abdeckung 13 bzw. ein Eintreffen von Pulverteilchen in die Öffnung 25, und schützt sowohl die Labyrinthdichtung als auch die Fluiddichtung.

Direkt in annähernd paralleler Richtung zur Messachse bzw. Messwelle 2 beschleunigte Pulverteilchen hoher kinetischer Energie können von der Fluiddichtung bzw. geometrischen Dichtung 7 nur mit hohen Fluid-Strömungsgeschwindigkeiten abgehalten werden. Allerdings würden derart hohe Strömungsgeschwingikeiten das im Messbehälter 1 gebildete Pulverfluid und die Messergebnisse beeinflussen. Der Schirm 8 löst dieses Problem.

Das somit mehrere Komponenten besitzende Dichtungssystem umfasst eine Dichtung mit feinen Kanälen bzw. dünnen Spalten 21 und der Schirm 8 schützt den Messmotor 4 und seine Lager bzw. die Steuereinheit 41 und die Auswerteeinheit 40 sowie die Normalkraftmesseinheit optimal, und selbst bei Versagen der Fluiddichtung werden in der mit feinen Kanälen ausgestatteten, geometrischen Feinstruktur Pulverstäube in dieser gefangen.

Durch das Einblasen der Luft in der Durchführung 7 bildet sich eine in den Messbehälter 1 gerichtete Luftströmung 47 aus, die die Sperrluftdichtung bildet. Bevorzugt sorgt eine feine geometrische Struktur im Bereich nahe der Außenseite der Wellendurchführung dafür, dass die Sperrluft bevorzugt in Richtung der Messkammer bzw. in den Messbehälter 1, und nicht an der feinen geometrischen Struktur vorbei nach außen strömt. Durch die Bewegung dieses Luftstroms 45 bildet sich je nach Geometrie eventuell zusätzlich ein Bernoullischer Unterdruck in der Dichtstruktur und es wird Luft von außerhalb der Messkammer 30 durch die Dichtstruktur nach innen angesaugt. Dieser nach innen die Dichtstruktur durchströmende Luftstrom 45 sorgt dafür, dass kein Aerosol zur Dichtstruktur gelangt und die Dichtstruktur somit nicht verschmutzt. Der Schirm 8 kann geometrisch so ausgeführt sein, dass der bernoullische Unterdruck maximiert wird, wie in Fig. 4 dargestellt ist.

Durch Entnehmen des Messkörpers 3 mit der Messwelle 2 und des Schirms 8 können die an der Messwelle 2 befindlichen Elemente der Dichtstruktur gereinigt werden. Gegebenenfalls können auf die Messwelle 2 auch Strukturen aufgebracht werden, die die geometrische Struktur des Dichtlagers ausbilden, z.B. in Form von Kunststoffteilen, die durch Drehen und/oder Fräsen oder Formgießen erstellt werden. Damit können die Lagerkomponenten, die von der Messwelle 2 mitbewegt werden, gewichtsmäßig leicht ausgeführt werden und z.B. auf die Messwelle 2 geschoben werden. Auch in die im Bereich der Durchführung 7 liegenden Bereiche der Abdeckung 13 können Strukturen ausgebildet oder angebracht werden.

Alternativ kann die Messwelle 2 direkt durch Drehen und/oder Fräsen mit den geometrischen Strukturen für die Dichtung versehen werden.

Gegebenenfalls können für unterschiedliche, z.B. agressive oder schwer fluidisierbare, Pulver auswechselbare Messkörper 3 und auswechselbare, an die Messkörßer 3 bzw. Pulver angepasste Dichtsysteme eingesetzt werden.

Es ist auch möglich, im Messbehälter 1 den Druckabfall über der Probe bzw. das Pulver 20 während der Fluidisierung zu messen, z.B. mittels Staurohrmethode.

Figur 2 zeigt ein Rheometer, dessen Messbehälter 1 von einer Temperierkammer 26 umgeben ist. Die Temperierkammer 26 umfasst eine Abdeckung 13 sowie einen Mantel und einen Bodenteil. Hier ist der Pulver- bzw. Staubschutz in die Abdeckung 13 der Temperierkammer 26 integriert. Die geometische Dichtung wird hier durch Zusammenspiel der Dichtgeometrie an der Messwelle 2 und an der Abdeckung 13 der Temperierkammer 26 bzw. Ausbildung und Verlauf des Spaltes 21 erstellt.

Das Pulver 20 im Messbehälter 1 kann in der Temperierkammer 26 unter definierten Umgebungsbedingungen untersucht werden, z.B. Feuchte, Temperatur. Es ist ein Vibrationsgenerator 19 angedeutet, der das im Messbehälter 1 befindliche Pulver 20 fluidisiert. Eine Fluidisierung wie in Fig. 1 dargestellt ist jedoch auch hier denkbar.

Der Schirm 8 an der Messwelle 2 besitzt im Querschnitt hier beispielsweise Trapezform und deckt die Öffnung 25 der Abdichtung bzw. Durchführung 7 ab. Die Abdichtung bzw. Durchführung 7 ist in vergleichbarer Weise wie bei der Ausführungsform gemäß Fig. 1 gestaltet.

Gegebenenfalls kann die Temperierkammer 26 einen Auslass 23 für Abluft mit einer Filteranordnung 23' enthalten, durch den das in die Temperierkammer 26 durch die Durchführung 7 eintretende Dichtfluid gemäß Pfeil 22 mit einer Saugeinheit 41 abgeleitet werden kann.

Die Figur 3a, b und c zeigen unterschiedliche Dichtungsgeometrien für Durchführungen 7 mit einer Luftführung entlang der Messwelle 2 in Zusammenspiel mit einem Staubschutzdeckel bzw. einer Abdeckung 13 eines Messbehälters 1 bzw. einer Temperierkammer 26.
Fig. 3 a zeigt einen an der Messachse befindlichen Schirm 8, der gemeinsam mit der Abdeckung 13 den Spalt 21 für die Fluiddichtung bildet. Als zusätzliche geometrische Feinstruktur ist hier (zumindest eine) feine geometrische Struktur, wie beispielsweise eine Nut 21 a in die Abdeckung 13 integriert.
Figur 3 b zeigt eine Anordnung mit einer Feinstruktur, die sowohl von der Abdeckung 13 als auch der Messwelle 2 getragen wird. Die von der Messwelle 2 getragenen geometrischen Strukturen 21 b bilden gemeinsam mit der umgebenden Struktur der Abdeckung 13 einen engen Spalt 21. Dieser kann direkt für die Zufuhr von Luft wie in Fig. 2 beschrieben verwendet werden, oder die Zufuhr erfolgt wie in Fig. 1 beschreiben mit einem hier nicht gezeichneten Kanal für die Fluidzufuhr.
Ist die geometrische Feinstruktur, wie z.B. eine Labyrinthdichtung, mit einem feinen verwinkeltem Spalt 21 ausgeführt, der durch Abdeckung 13 und die Dichtstruktur an der Messwelle 2 bildet wird, so muss die Abdeckung 13 zweiteilig ausgeführt sein und sich um die Messwelle entsprechend der Pfeile schließen lassen, um einen Wechsel der Messwellen 2 und damit der Messkörper 3 zu ermöglichen.
Figur 3 c zeigt eine Ausführungsform der Duchführung 7, die den Schirm 8 durch Verjügung der Messwelle 2 an der Durchführung 7 bildet. Allenfalls kann hier die Fluiddichtung durch Fluidzuführungen 12 an der Abdeckung 13 gebildet werden. Auch hier muss die Abdeckung 13 mehrteilig ausgeführt werden, um die Messwelle 2 entnehmen zu können. Der Schirm 8 ist von der Messwelle 2 gebildet.
Figur 4 zeigt eine Anordnung, bei der der Schirm 8 geometrisch so ausgeführt ist, dass der bernoullische Unterdruck maximiert wird. Der gerichtete Luftstrom 45 sorgt zusätzlich zur geometrischen Dichtung dafür, dass kein Aerosol zur Dichtstruktur gelangt und die Dichtstruktur somit nicht verschmutzt. Die Führung des gerichteten Luftstroms 45 im annähernd rechten Winkel zur Öffnung 25 der berührungslosen Durchführung der Messwelle 2 bzw. zu dem zwischen der Unterseite der Abdeckung 13 und dem Schirm 8 ausgebildeten Spalt 46 maximiert vor dem Spalt 46 den Unterdruck und somit den Bernoulli-Effekt und führt zu einer starken gerichteten Strömung der die Durchführung 7 durchströmenden Luft in die Messkammer bzw. den Messbehälter 1 hinein. Die Zufuhr des Luftstroms 45 erfolgt über einen in der Abdeckung 13 ausgebildeten Kanal 50.

Die gerundete Fläche 47 am oberen Endbereich des Schirms 8 unterstützt die Luftströmung 45 bzw. die Ausbildung eines Unterdrucks.

Die Kombination einer geometrischen Dichtung mit einer Sperrluftdichtung ist optimal für ein Zurückhalten des Feinanteils des Aerosols. Die eingesetzte Dichtung besitzt einen Spalt von nur wenigen Zehntel-Millimetern zwischen der Abdeckung des Behälters bzw. seiner Staubschutzhaube und dem Messsystemschaft bzw. der Messwelle 2. Die Sperrluftdichtung umfasst einen Spalt bzw. eine Zufuhröffnung für ein Fluid an der Innenseite der Abdeckung oder der Staubschutzhabe bzw. der Abdeckung des Hüllgehäuses, in den bzw. die ein nach innen in den Behälter gerichteter, vorzugsweise gleichmäßiger, Luftstrom strömt bzw. aufrecht erhalten wird, der den Feinanteil ständig vom Spalt bzw. der Zufuhröffnung und der Messwelle 2 wegbläst, aber trotzdem die Messung nur vernachlässigbar beeinflusst.

Bevorzugt wird die geometrische Dichtung bzw. die feine labyrinthartige Struktur motorseitig entlang bzw. oberhalb der Durchführung der Messwelle, d.h. auch oberhalb einer Fluidzufuhr für die Fluiddichtung ausgeführt, wobei die Fluiddichtung bereits den Großteil des Feinanteils des Pulvers in das Innere des Messbehälters rückführt.

Prinzipiell muss die Verzahnung der geometrischen Struktur nicht unbedingt nach Art einer Labyrinthdichtung ineinander greifen und keine "klassische" Labyrinthdichtung wie in Fig. 3b dargestellt ausbilden. Wenn Luft bzw. Fluid durch den Spalt der Dichtung vom Aufnahmeraum bzw. vom Inneren des Messbehälters 1 nach außen strömt, soll dem Luftstrom dabei ein möglichst hoher Widerstand geboten werden. Die Kombination aus Fluiddurchfluss und Spaltweite muss passend gewählt werden, sodass die Geschwindigkeit des Spülgases eine derart hohe kinetische Energie aufweist um das Aerosol und die darin enthaltenen Pulverpartikel zuverlässig aus dem Bereich der Wellendurchführung zu halten. Ein dünner Spalt (wenige Millimeter, bevorzugt wenige Zehntel-Millimeter) und einer Spülgasgeschwindigkeit von einigen m/s sorgt für diesen hohen Widerstand, vor allem bei Pulvern einer Teilchengröße von einigen Nanometern bis zu Teilchen in der Größenordnung der Spaltweite. Der Fachmann kann abhängig von den Rheometereigenschaften einen optimalen Kompromiss aus Spaltweite und Spülgasgeschwindigkeit wählen. Zusätzliche Nuten und/oder Absätze im Querschnitt der Abdichtung und/oder an der Messwelle 2 - siehe Fig. 3a, 3b, 3c - erschweren dem Aerosol das Durchströmen der Wellendurchführung und verhindern zudem das Ausbilden einer laminaren Strömung aus der Messkammer. Bevorzugt wird die Spülgaszuleitung in die Durchführung derart gewählt, sodass der Gaseintritt unterhalb der geometrischen Dichtung bzw. der Nuten erfolgt und die eintretende Luft bevorzugt in Richtung Messkammer strömt. Die Luftteilchen bzw. das Aerosol können nicht gerade bzw. ungehindert durch den Spalt aus der Messkammer strömen, sondern werden durch sich ausbildende Wirbel in den Nuten zusätzlich blockiert. Es handelt sich um eine geometrische Dichtung, weil das Fluidisierungsmedium, das den Feinanteil des Pulvers als Schwebstoff enthält, durch diese Art von Durchgang nicht bzw. nur schwer durchtreten kann.

Derartige geometrische Sperren sind vor allem für das Zurückhalten von Teilchen mit einer Partikelgröße sehr viel kleiner als die Durchführungs-Spaltweite geeignet.

Ein Splashguard bzw. Schirm wird eingesetzt, der die Öffnung für die Durchführung der Messwelle durch die Abdeckung des Messehälters abdeckt und von der Messwelle 2 oder dem Messbehälter 1 getragen ist. Dieser Schirm 8 schützt den Spalt der Dichtung bzw. die Durchführung auch vor aus dem Pulverbett geschleuderte großen, schweren Pulverteilchen mit hoher gerichteter kinetischer Energie.

Erfindungsgemäß wird eine Kombination von beiden geometrischen Komponenten, nämlich von einem Schirm 8 und eine einen dünnen Spalt aufweisende Dichtung gegebenenfalls noch in Kombination mit einem zumindest teilweise fluidgedichteten Hüllgehäuse rund um die Messwelle bzw. den Messbehälter verwendet.

Die Tempeprierung der Pulverprobe kann beispielsweise durch eine Klimakammer, deren Durchführung die vorgeschlagene Kombination aus Fluiddichtung und geometrischer Pulversperre ausbildet, oder über Temperierelemente direkt am Messbehälter 1 und/oder an der Abdeckung 13 des Messbehälters 1 oder auch durch Zuführung von temperierten, feuchten Gasen für die Fluidisierung ausgeführt werden.

Wesentlich ist die Kombination einer Fluiddichtung mit zusätzlichen geometrischen Mitteln bzw. Dichtstrukturen. Die Fluiddichtung ist im wesentlichen ausschließlich durch den einfachen Durchführungsspalt für die Messwelle 2 mit einer Durchströmung gebildet. Um das Durchfließen des Pulvers bzw. von Staub und Teilchen mit hoher kinetischer Energie sicher zu verhindern, wird diese Fluiddichtung mit weiteren geometrischen Mitteln kombiniert. Dies kann einerseits der Schirm 8 sein, welcher auch die Funktion besitzt, die Einströmung in Richtung Probe bzw. Messkörper 3 zu begrenzen, oder andererseits die Feinstruktur mit Nuten bis hin zur Geometrie einer Labyrinthdichtung.

Für ein Pulverrheometer ist es erforderlich, die Fluiddichtung in Kombination mit weiteren geometrischen Dichtanordnungen verwendet werden. Bevorzugt ist eine Kombination aus allen drei beschriebenen Varianten umfassend den Schirm 8 für die hochenergetischen, großen Teilchen sowie eine Feinstruktur für die kleineren Teilchen und Staubpartikel und die Fluiddichtung. Damit wird sichergestellt, dass kein Staub in die empfindlichen Rheometer - in den Zeichnungen mit dem Messmotor angedeutet - gelangt.

Die Feinstruktur kann entweder oberhalb oder unterhalb der Fluidzuleitung, vorzugsweise oberhalb der Fluidzuleitung wie in Fig. 1 und Fig. 4 dargestellt, angeordnet sein.

## Patentansprüche

1. Drehrheometer zur Vermessung von pulvrigen oder granularen Materialien, umfassend einen Messbehälter (1) zur Aufnahme des Messguts (20), eine Abdeckung (13) für den Messbehälter (1), einen von einer Messwelle (2) gehaltenen Messkörper (3), wobei der Messkörper (3) und der Behälter (1) relativ zueinander rotierbar sind, wobei die Messwelle (2) berührungslos durch die Abdeckung (13) geführt ist und eine außerhalb des Messbehälters (1) angeordnete Auswerteeinheit (40) zur Auswertung der von der Messwelle (2) abgenommenen Messwerte, wobei zur Abdichtung des Lagerspaltes oder der Durchführung (7) der Messwelle (2) in der oder durch die Abdeckung (13) in den Messbehälter (1) sowohl eine Fluiddichtung mit einer Dichtfluidzuleitung als auch zumindest eine mit dieser Fluiddichtung zusammenwirkende geometrische Dichtung als Pulversperre (8) vorgesehen sind, wobei als erste geometrische Dichtung von der Messwelle (2) innerhalb des Messbehälters (1) ein Schirm (8) getragen ist, dessen Flächenerstreckung senkrecht zur Messwelle (2) die Öffnung (25) der Durchführung (7) allseitig überragt und
- (a) zumindest eine Ausströmöffnung, vorzugsweise mehrere Ausströmöffnungen, der Dichtfluidzuleitung auf der Unterseite der Abdeckung (13) im Nahbereich der Durchführung (7) der Messwelle (2) und oberhalb des Schirms (8) liegt oder
- (b) ein Lufteinlass (10) unterhalb einer innerhalb der Abdeckung (1) gelegenen zweiten geometrischen Dichtung in die Durchführung (7) mündet. und eine Einheit zur Fluidisierung des Messguts (20) im Messbehälter (1) vorgesehen oder an diesen angeschlossen ist.

2. Drehrheometer nach Anspruch 1, **dadurch gekennzeichnet, dass** die Messwelle (2) und die Abdeckung (13) gemeinsam als zweite geometrische Dichtung eine Dichtfeinstruktur, vorzugsweise eine Labyrinthdichtung, ausbilden.

3. Drehrheometer nach der Alternative (b) von Anspruch 1 oder nach Anspruch 2, **dadurch gekennzeichnet, dass** die Fluiddichtung eine an den Messbehälter (1) angeschlossene Einrichtung (41) zur Erzeugung eines Unterdrucks umfasst, mit der durch den Spalt (21) der Durchführung (7) zwischen der Messwelle (2) und der Abdeckung (13) im Bereich unterhalb der zweiten geometrischen Dichtung Außenluft oder Dichtgas in den Messbehälter (1) angesaugt wird.

4. Drehrheometer nach einem der Ansprüche 2 bis 3, **dadurch gekennzeichnet**, das, wenn zumindest eine Ausströmöffnung, vorzugsweise mehrere Ausströmöffnungen, der Dichtfluidzuleitung auf der Unterseite der Abdeckung (13) im Nahbereich der Durchführung (7) der Messwelle (2) und oberhalb des Schirms (8) liegt, als Fluiddichtung in den Bereich der Durchführung (7) zwischen der zweiten geometrischen Dichtung und dem Inneren des Messbehälters (1), insbesondere in die Abdeckung (13) oder in die Durchführung (7) der Messwelle (2), eine Zufuhrleitung (12) zur Zufuhr eines den Gasdruck im Messbehälter (1) überschreitenden Druck aufweisenden Dichtgases einmündet.

5. Drehrheometer nach einem der Ansprüche 1 bis 4, **dadurch gekennzeichnet, dass** der Schirm (8) in einem Abstand von der Durchführung (7) angeordnet, welcher Abstand kleiner als der Durchmesser, vorzugsweise der Radius, der Durchführung (7) ist oder dass der Schirm (8) zumindest teilweise in der Durchführung (7) gelegen ist.

6. Drehrheometer nach einem der Ansprüche 1 bis 5, **dadurch gekennzeichnet, dass** sich der Schirm (8) parallel zur Öffnung (25) der Durchführung (7) erstreckt.

7. Drehrheometer nach einem der Ansprüche 1 bis 6, **dadurch gekennzeichnet**, das, wenn zumindest eine Ausströmöffnung, vorzugsweise mehrere Ausströmöffnungen, der Dichtfluidzuleitung auf der Unterseite der Abdeckung (13) im Nahbereich der Durchführung (7) der Messwelle (2) und oberhalb des Schirms (8) liegt, das Drehrheometer derart ausgebildet ist, dass das Dichtfluid durch die Ausströmöffnung unter Druck in den Messbehälter (1) einströmbar oder durch Ausbildung eines Unterdrucks im Messbehälter (1) in diesen einsaugbar ist

8. Drehrheometer nach einem der Ansprüche 1 bis 7, **dadurch gekennzeichnet**, das, wenn zumindest eine Ausströmöffnung, vorzugsweise mehrere Ausströmöffnungen, der Dichtfluidzuleitung auf der Unterseite der Abdeckung (13) im Nahbereich der Durchführung (7) der Messwelle (2) und oberhalb des Schirms (8) liegt, die Ausströmöffnung von einem die Messwelle (2) umgebenden Spalt (46) zwischen der Unterseite der Abdeckung (13) und dem Schirm (8) gebildet ist.

9. Drehrheometer nach einem der Ansprüche 1 bis 8, **dadurch gekennzeichnet, dass** als weitere geometrische Dichtung Nuten (21a) in der Abdeckung (13) und/oder der Messwelle (2) ausgebildet sind.

## Claims

1. Rotational rheometer for measuring powdery or granular materials, comprising a measuring container (1) for receiving the material to be measured (20), a cover (13) for the measuring container (1), a measuring body (3) held by a measuring shaft (2), wherein the measuring body (3) and the container (1) can be rotated relative to one another, wherein the measuring shaft (2) is guided through the cover (13) without contact, and an evaluation unit (40) arranged outside the measuring container (1) for evaluating the measured values taken from the measuring shaft (2), wherein for sealing the bearing gap or the passage (7) of the measuring shaft (2) in or through the cover (13) into the measuring container (1) both a fluid seal with a sealing fluid supply line and at least one geometric seal interacting with this fluid seal are provided as a powder barrier (8), wherein a screen (8) is supported as the first geometric seal of the measuring shaft (2) inside the measuring container (1), the surface extension of said screen protruding over the opening (25) of the passage (7) on all sides and perpendicular to the measuring shaft (2) and
a) at least one outflow opening, preferably several outflow openings, of the sealing fluid supply line is located on the underside of the cover (13) in the vicinity of the passage (7) of the measuring shaft (2) and above the screen (8) or
b) an air inlet (10) opens into the passage (7) below a second geometric seal located inside the cover (1),
and a unit for fluidising the material to be measured (20) is provided in the measuring container (1) or is connected to this.

2. Rotational rheometer according to claim 1, **characterised in that** the measuring shaft (2) and the cover (13) together form a fine sealing structure as a second geometric seal, preferably a labyrinth seal.

3. Rotational rheometer according to alternative (b) of claim 1 or according to claim 2, **characterised in that** the fluid seal comprises an apparatus (41) connected to the measuring container (1) for generating a negative pressure with which outside air or sealing gas is sucked into the measuring container (1), through the gap (21) in the passage (7) between the measuring shaft (2) and the cover (13) in the area below the second geometric seal.

4. Rotational rheometer according to any one of claims 2 to 3, **characterised in that**, when at least one outflow opening, preferably several outflow openings, of the sealing fluid supply line is located on the underside of the cover (13) in the vicinity of the passage (7) of the measuring shaft (2) and above the screen (8), a supply line (12) opens as a fluid seal into the area of the passage (7) between the second geometric seal and the interior of the measuring container (1), in particular into the cover (13) or into the passage (7) of the measuring shaft (2), for the supply of a sealing gas having a pressure exceeding the gas pressure in the measuring container (1).

5. Rotational rheometer according to any one of claims 1 to 4, **characterised in that** the screen (8) is arranged at a distance from the passage (7), which distance is smaller than the diameter, preferably the radius, of the passage (7), or **in that** the screen (8) is at least partially located in the passage (7).

6. Rotational rheometer according to any one of claims 1 to 5, **characterised in that** the screen (8) extends parallel to the opening (25) of the passage (7).

7. Rotational rheometer according to any one of claims 1 to 6, **characterised in that**, if at least one outflow opening, preferably several outflow openings, of the sealing fluid supply line is located on the underside of the cover (13) in the vicinity of the passage (7) in the measuring shaft (2) and above the screen (8), the rotational rheometer is designed in such a way that the sealing fluid can flow into the measuring container (1) through the outflow opening under pressure or can be sucked into the measuring container (1) by creating a negative pressure therein

8. Rotational rheometer according to any one of claims 1 to 7, **characterised in that**, if at least one outflow opening, preferably several outflow openings, of the sealing fluid supply line is located on the underside of the cover (13) in the vicinity of the passage (7) of the measuring shaft (2) and above the screen (8), the outflow opening is formed by a gap (46) surrounding the measuring shaft (2) between the underside of the cover (13) and the screen (8).

9. Rotational rheometer according to any one of claims 1 to 8, **characterised in that** grooves (21a) are formed in the cover (13) and/or the measuring shaft (2) as a further geometric seal.

## Revendications

1. Rhéomètre rotatif pour mesurer des matériaux pulvérulents ou granulaires, comprenant un récipient de mesure (1) destiné à recevoir la matière à mesurer (20), un couvercle (13) pour le récipient de mesure (1), un corps de mesure (3) maintenu par un arbre de mesure (2), dans lequel le corps de mesure (3) et le récipient (1) peuvent tourner l'un par rapport à l'autre, dans lequel l'arbre de mesure (2) est conduit sans contact au travers du couvercle (13) et une unité d'évaluation (40) disposée à l'extérieur du récipient de mesure (1) pour évaluer les valeurs de mesure issues de l'arbre de mesure (2), dans lequel pour l'étanchéité de l'intervalle de palier ou pour le passage (7) de l'arbre de mesure (2) dans le ou au travers du couvercle (13) sont prévus dans le récipient de mesure (1) à la fois un joint étanche aux fluides avec une conduite d'acheminement de fluide d'étanchéité et également au moins un joint géométrique interagissant avec ce joint étanche aux fluides en tant que barrière aux poudres (8), dans lequel en tant que premier joint géométrique de l'arbre de mesure (2) à l'intérieur du récipient de mesure (1) est porté un écran (8) dont l'étendue superficielle surmonte de tous côtés perpendiculairement à l'arbre de mesure (2) l'ouverture (25) du passage (7) et
(a) au moins une ouverture d'évacuation, de préférence plusieurs ouvertures d'évacuation de la conduite d'acheminement de fluide d'étanchéité sur le côté inférieur du couvercle (13) se situe à proximité du passage (7) de l'arbre de mesure (2) et au-dessus de l'écran (8) ou
(b) une entrée d'air (10) en-dessous d'un second joint géométrique situé à l'intérieur du couvercle (1) débouche dans le passage (7),
et une unité pour fluidiser le matériau à mesurer (20) est prévue dans le récipient de mesure (1) ou est raccordée à celui-ci.

2. Rhéomètre rotatif selon la revendication 1, **caractérisé en ce que** l'arbre de mesure (2) et le couvercle (13) forment conjointement en tant que second joint géométrique une microstructure étanche, de préférence un joint en labyrinthe.

3. Rhéomètre rotatif selon l'alternative (b) selon la revendication 1 ou selon la revendication 2, **caractérisé en ce que** le joint étanche aux fluides comprend un dispositif (41) raccordé au récipient de mesure (1) pour générer une pression négative, avec lequel au travers de la fente (21) du passage (7) entre l'arbre de mesure (2) et le couvercle (13) dans la zone en-dessous du second joint géométrique, de l'air extérieur ou un gaz d'étanchéité est aspiré dans le récipient de mesure (1).

4. Rhéomètre rotatif selon l'une quelconque des revendications 2 à 3, **caractérisé en ce que**, lorsqu'au moins une ouverture d'évacuation, de préférence plusieurs ouvertures d'évacuations de la conduite d'acheminement de fluide d'étanchéité se situe(nt) sur le côté inférieur du couvercle (13) à proximité du passage (7) de l'arbre de mesure (2) et au-dessus de l'écran (8), en tant que joint étanche aux fluides dans la zone du passage (7) entre le second joint géométrique et l'intérieur du récipient de mesure (1), en particulier dans le couvercle (13) ou dans le passage (7) de l'arbre de mesure (2), une conduite d'acheminement (12) débouche pour acheminer un gaz d'étanchéité présentant une pression dépassant la pression de gaz dans le récipient de mesure (1).

5. Rhéomètre rotatif selon l'une quelconque des revendications 1 à 4, **caractérisé en ce que** l'écran (8) est disposé dans un intervalle du passage (7), lequel intervalle est inférieur au diamètre, de préférence au rayon du passage (7) ou **en ce que** l'écran (8) est placé au moins en partie dans le passage (7).

6. Rhéomètre rotatif selon l'une quelconque des revendications 1 à 5, **caractérisé en ce que** l'écran (8) s'étend parallèlement à l'ouverture (25) du passage (7).

7. Rhéomètre rotatif selon l'une quelconque des revendications 1 à 6, **caractérisé en ce que**, lorsqu'au moins une ouverture d'évacuation, de préférence plusieurs ouvertures d'évacuation de la conduite d'acheminement de fluide d'étanchéité se situe(nt) sur le côté inférieur du couvercle (13) à proximité du passage (7) de l'arbre de mesure (2) et au-dessus de l'écran (8), le rhéomètre rotatif est formé de telle sorte que le fluide d'étanchéité peut être acheminé au travers de l'ouverture d'évacuation sous pression dans le récipient de mesure (1) ou peut être aspiré dans celui-ci en formant une pression négative dans le récipient de mesure (1)

8. Rhéomètre rotatif selon l'une quelconque des revendications 1 à 7, **caractérisé en ce que**, lorsqu'au moins une ouverture d'évacuation, de préférence plusieurs ouvertures d'évacuation de la conduite d'acheminement de fluide d'étanchéité se situe(nt) sur le côté inférieur du couvercle (13) à proximité du passage (7) de l'arbre de mesure (2) et au-dessus de l'écran (8), l'ouverture d'évacuation est formée par une fente (46) entourant l'arbre de mesure (2) entre le côté inférieur du couvercle (13) et l'écran (8).

9. Rhéomètre rotatif selon l'une quelconque des revendications 1 à 8, **caractérisé en ce que** des rainures (21a) sont formées dans le couvercle (13) et/ou l'arbre de mesure (2) en tant qu'autre joint géométrique.
